# EUROPEAN PATENT SPECIFICATION

(11) **EP 2 525 227 B1**
(45) Date of publication and mention of the grant of the patent: **19.11.2014**
(21) Application number: 12168401.3
(22) Date of filing: 16.05.2012
(51) Int. Cl.: G01N 33/574

(54) **A method for detecting pancreatic cancer using the serological marker ULBP2**
Verfahren zur Erkennung von Bauchspeicheldrüsenkrebs unter Verwendung des serologischen Markers ULBP2
Procédé de détection du cancer du pancréas en utilisant le marquer sérologique ULBP2

(30) Priority: 19.05.2011 TW 100117502
(43) Date of publication of application: 21.11.2012
(73) Proprietor: Chang Gung University (a university of Taiwan), Kwei-Shan Tao-Yuan (TW)
(72) Inventor: Yu, Jau-Song, Tao-Yuan (TW); Chang, Ya-Ting, Taichung City (TW); Wu, Chih-Ching, Pingtung City, Pingtung County (TW); Shyr, Yi-Ming, Taipei City (TW); Chang, Yu-Sun, 244 Linkou Shiang, Taipei County (TW)
(74) Representative: Lang, Christian

(56) References cited:
- WO-A2-2011/050328
- BÜNGER S ET AL: "Serum biomarkers for improved diagnostic of pancreatic cancer: a current overview", JOURNAL OF CANCER RESEARCH AND CLINICAL ONCOLOGY, SPRINGER, BERLIN, DE, vol. 137, no. 3, 31 December 2010 (2010-12-31), pages 375-389, XP019881018, ISSN: 1432-1335, DOI: 10.1007/S00432-010-0965-X
- ONDA ET AL: "A Novel Secreted Tumor Antigen with a Glycosylphosphatidylinositol-Anchored Structure Ubiquitously Expressed in Human Cancers", BIOCHEMICAL AND BIOPHYSICAL RESEARCH COMMUNICATIONS, ACADEMIC PRESS INC. ORLANDO, FL, US, vol. 285, no. 2, 13 July 2001 (2001-07-13) , pages 235-243, XP005100770, ISSN: 0006-291X, DOI: 10.1006/BBRC.2001.5149
- P. WROBEL ET AL: "Lysis of a Broad Range of Epithelial Tumour Cells by Human gamma delta T Cells: Involvement of NKG2D ligands and T-cell Receptor- versus NKG2D-dependent Recognition", SCANDINAVIAN JOURNAL OF IMMUNOLOGY, vol. 66, no. 2-3, 1 August 2007 (2007-08-01), pages 320-328, XP55036582, ISSN: 0300-9475, DOI: 10.1111/j.1365-3083.2007.01963.x
- YA-TING CHANG ET AL: "Secretome-Based Identification of ULBP2 as a Novel Serum Marker for Pancreatic Cancer Detection", PLOS ONE, vol. 6, no. 5, 20 May 2011 (2011-05-20), page e20029, XP55036528, ISSN: 1932-6203, DOI: 10.1371/journal.pone.0020029

## Description

### FIELD OF THE INVENTION

Embodiments relate to a serological marker for detecting pancreatic cancer and a method for using the serological marker, especially to a serological marker with high sensitivity and specificity for detecting pancreatic cancer and a method for using the serological marker.

### BACKGROUND OF THE INVENTION

Pancreatic cancer is respectively ranked fourth and tenth among cancer-related mortality in United State and Taiwan and shows increased mortality these years. Because pancreas is anatomically located in a deeper site and the apparent symptoms of the pancreatic cancer is late onset, less than 8% of pancreatic cancer patients are diagnosed at the localized stage and can be surgically cured. More than 50% of diagnosed pancreatic cancer patients have exhibited distant metastasis, and the 5-year survival rate of which is less than 5%.

Bünger S et al. (J Cancer Res Clin Oncol. 137(3):375-89. Epub 2010 Dec 31. Serum biomarkers for improved diagnostic of pancreatic cancer: a current overview) gave an overview on serum biomarkers with screening potential for pancreatic cancer. The authors retrieved 1180 relevant articles and selected 43 papers from the PUBMED database, in which the diagnostic performance of 41 individual serum markers for detecting pancreatic cancer were compared and evaluated. This analysis disclosed methods for detecting pancreatic cancer in a blood specimens obtained from a testee using individual serum markers such as MIC-1, PAM4, OPN, HSP27, TPS, TSGF, CAM17.1, PF4, and CEACAM1, which showed superior values for diagnostic performance. As most of these serum markers were tested in a small sample cohort, further standardized validation studies using multiplex assays in large and muticenter sample cohort(s) are needed to verify their utilities in clinical settings. As none of the 43 papers mentioned ULBP2 (the target serum marker in the present application) as a serum marker for detecting pancreatic cancer, the present invention may therefore be regarded as the provision of an alternative serological biomarker for detecting pancreatic cancer.

Onda et al. (Biochem Biophys Res Commun. 2001 Jul 13;285(2):235-43. A novel secreted tumor antigen with a glycosylphosphatidylinositol-anchored structure ubiquitously expressed in human cancers) reported that ALCAN (which is a synonym for ULBP2) may be a potential target for cancer diagonosis because its mRNA could be detected in cell lines from various human cancer types (lung, breast, colon, prostate, neuroblastma, glioma, leukemia, T-cell lyphoma and chondrosarcoma) but not (or barely) in several normal organs. However, the levels of ALCAN expression and secretion vary drastically in different cell lines, and more importantly, this paper is completely silent about the relation between ALCAN and pancreatic cancer.

WO 2011/050328 A2 (Publication Date:28.04.2011. ASSESSMENT OF SOLID TUMOR BURDEN) disclosed a method to assess solid tumor burden on the basis of measuring expression levels of one or both of an activating Natural Killer (NK) cell receptor ligand(s) (NKG2D ligands) on peripheral blood cells in a blood specimens obtained from a testee (claim 1) and mentioned ULBP2 as one instance for the NKG2D ligand (claim 2) and pancreatic cancer as one example for dozens of the solid tumor types listed (claim 4). However, this article only provided experimental data and gave the examples regarding three NKG2D ligands (MICA, MICB and ULBP1) (claim 3) using specimens from patients with 4 cancer types (glioblastoma multiforme, hepatocellular carcinoma, prostate cancer or breast cancer)(claim 5). Therefore, claims 2 and 4 just represent two independent "wish lists" containing a variety of biomarkers and cancer types, and the particular combination of ULBP2 and pancreatic cancer is not disclosed in an enabling way. In addition, this article focuses on measuring the NKG2D ligand levels on peripheral blood cells in a blood specimens, which differ distinctly from that (i.e. the serum specimens) of the present invention. Moreover, this article did not disclose the overexpression of any of the NKG2D ligands in cancer cells from any of the tissue specimens examined.

Wrobel et al. (Scand J Immunol. 2007 Aug-Sep;66(2-3):320-8. Lysis of a broad range of epithelial tumour cells by human gamma delta T cells: involvement of NKG2D ligands and T-cell receptor-versus NKG2D-dependent recognition) showed the surface expression of NKG2D ligands (MICA/B and ULBP-1-3) on various epithelial cancer cell lines including pancreatic tumour cell lines PancTu1 and Pt45-P1. The proteolytically released of ULBP2 from the surface of tumour cells has also been mentioned in the discussion section of this article. Nonetheless, this article focuses on investigating the role of NKG2D ligands in the T-cell-based immunotherapy of cancer using the cell culture model and is absolutely silent about diagnostic aspects.

Current approaches for pancreatic cancer diagnosis are mainly based on imaging methods, such as abdominal sona or high-resolution computed tomography scan, and sometimes might combine with an invasive endoscopy or a magnetic resonance cholangiopancreatography to increase detection efficiency. However, the volume of pancreatic cancer in early stage is too small to be detected by the imaging method, which greatly increases the difficulty in detecting pancreatic cancer. With the development of molecular diagnosis and tumor biology, a serological marker, carbohydrate antigen 19-9 (CA 19-9), is

widely applied in detecting pancreatic cancer. However, as current knowledge, CA 19-9 has few disadvantages in pancreatic cancer detection, such as the insufficient sensitivity and specificity and the unable detection of pancreatic cancer in early stage. Therefore, developing other serological markers to overcome above-mentioned disadvantages of CA19-9 and increase the detection efficiency will be an important issue to improve the management of pancreatic cancer. This is achieved by the subject matter of claim 1.

### SUMMARY OF THE INVENTION

According to one aspect of an embodiment of the invention, a serological marker for detecting a pancreatic cancer with high sensitivity and specificity comprising at least an UL16 binding protein 2 (ULBP2) is provided. The ULBP2 is highly expression in pancreatic tumor tissues, and is significant increased in serum of a pancreatic cancer patient than in a normal healthy person. The ULBP2 is also significant increased in pancreatic cancer than in gastric cancer (GC), nasopharyngeal carcinoma cancer (NPC) and colorectal carcinoma cancer (CRC). Therefore, the ULBP2 is capable to be a serological marker for efficiently detecting pancreatic cancer by comparing its levels in the blood samples isolated from a patient and a normal healthy person.

According to another aspect of an embodiment of the invention, a bead-based immunoassay at least using an UL16 binding protein 2 (ULBP2) as a serological marker is used to detect pancreatic cancer and has exhibited an excellent sensitivity. The limitation of the bead-based immunoassay in detecting the pancreatic cancer is 3.91pg/ml.

In a further embodiment of the invention, ULBP2 is combined with a carbohydrate antigen 19-9 (CA 19-9) to improve the detection efficiency. The ULBP2 also has ability to detect the pancreatic cancer at early stage and has more precise detection effect than CA 19-9.

The above objects and advantages of the present invention will become more readily apparent to those ordinarily skilled in the art after reviewing the following detailed descriptions and accompanying drawings in which:

### BRIEF DESCRIPTIONS OF THE DRAWINGS

Fig. 1 is a standard calibration curve of a serological marker for detecting pancreatic cancer by using a bead-based immunoassay.
Fig. 2 is a receiver operating characteristic curve of the ULBP2 and CA19-9 in detecting pancreatic cancer.
Fig. 3A shows the detection efficiency of ULBP2, CA19-9 and ULBP2 combined with CA19-9 in detecting the pancreatic cancer of T1/T2 stage of the TNM classification.
Fig. 3B shows the detection efficiency of ULBP2, CA19-9 and ULBP2 combined with CA19-9 in detecting the pancreatic cancer of N0 stage of the TNM classification.
Fig. 3C shows the detection efficiency of ULBP2, CA19-9 and ULBP2 combined with CA19-9 in detecting the pancreatic cancer of I-II stage of the overall stage.
Fig. 4 shows the immunohistochemistry assay of the cancer tissue biopsies from 67 pancreatic cancer patients shows that ULBP2 staining is positive in all biopsies (100%), and the expression of ULBP2 is more significant in the cancer tissue than in the non-cancerous tissues.

### DETAILED DESCRIPTION OF THE PREFERRED EMBODIMENT

Embodiment 1: screening and selecting a serological marker for detecting the pancreatic cancer

Proteins secreted from two pancreatic cancer cell lines PANC-1 and MIA PaCa-2, which were collected by incubating the cultured cells in serum-free medium for 24 hr (this medium is thereafter defined as conditioned medium), are systematically identified by one-dimensional SDS-PAGE in conjunction with nano-LC-MS/MS (the GeLC-MS/MS approach). This method identified a total of 1812 non-redundant proteins from the conditioned medium of the two cell lines. The transcriptional expression of each identified protein in the pancreatic cancer tissues was further analyzed according to a public doamin transcriptomic information of the pancreatic cancer tissues (National Center for Biotechnology Information (NCBI) Gene Expression Omnibus). In this transcriptome dataset, pancreatic ductal cells respectively isolated from 25 healthy donors and 24 pancreatic cancer patients are subjected to an array-based analysis to identify the genes whose message RNA (mRNA) levels are higher expressed in the pancreatic cancer patients than those in the healthy donors. By integrating this transcriptome dataset and the secreted protein database of the two pancreatic cancer cell lines generated as described above, 30 pancreatic cancer cell secreted proteins exhibited at least two-fold higher mRNA expression levels in the pancreatic ductal cells from cancer patients than from the healthy donors. Eleven out of the 30 proteins had been reported to be over-expressed in the tissue biopsy of pancreatic cancer by previous studies. Among the other 19 proteins without any references related to pancreatic cancer, UL16 binding protein 2 (ULBP2) is selected as a serological candidate marker for detecting pancreatic cancer in the present invention.

The ULBP2 in the present embodiment has an amino acid sequence (SEQ ID NO:1) shown as following:

Met Ala Ala Ala Ala Ala Thr Lys Ile Leu Leu Cys Leu Pro Leu Leu Leu Leu
Leu Ser Gly Trp Ser Arg Ala Gly Arg Ala Asp Pro His Ser Leu Cys Tyr Asp Ile
Thr Val Ile Pro Lys Phe Arg Pro Gly Pro Arg Trp Cys Ala Val Gln Gly Gln Val
Asp Glu Lys Thr Phe Leu His Tyr Asp Cys Gly Asn Lys Thr Val Thr Pro Val
Ser Pro Leu Gly Lys Lys Leu Asn Val Thr Thr Ala Trp Lys Ala Gln Asn Pro Val
Leu Arg Glu Val Val Asp Ile Leu Thr Glu Gln Leu Arg Asp Ile Gln Leu Glu
Asn Tyr Thr Pro Lys Glu Pro Leu Thr Leu Gln Ala Arg Met Ser Cys Glu Gln
Lys Ala Glu Gly His Ser Ser Gly Ser Trp Gln Phe Ser Phe Asp Gly Gln Ile Phe
Leu Leu Phe Asp Ser Glu Lys Arg Met Trp Thr Thr Val His Pro Gly Ala Arg
Lys Met Lys Glu Lys Trp Glu Asn Asp Lys Val Val Ala Met Ser Phe His Tyr
Phe Ser Met Gly Asp Cys Ile Gly Trp Leu Glu Asp Phe Leu Met Gly Met Asp
Ser Thr Leu Glu Pro Ser Ala Gly Ala Pro Leu Ala Met Ser Ser Gly Thr Thr Gln
Leu Arg Ala Thr Ala Thr Thr Leu Ile Leu Cys Cys Leu Leu Ile Ile Leu Pro Cys

One ordinary skill in the art understands that any amino acid replacement by an amino acid with similar characteristic will cause a little variation in the original SEQ ID NO:1. However, a sequence has similarity more than 95% to SEQ ID NO:1 is considered as a serological marker for detecting pancreatic cancer that can be used in the embodiment.

### Embodiment 2: the expression of the serological marker ULBP2 in pancreatic cancer tissues

Immunohistochemistry assay: In the embodiment, a goat anti-ULBP2 antibody is applied. A tissue biopsy is isolated and heated in a 0.01 M citric acid buffer (pH 6.0). A blocking buffer is added and reacted at room temperature for 5 minutes. The tissue biopsy is reacted with the anti-ULBP2 antibody (1:20 dilution) at 4°C for 16 hours. Then, the tissue biopsy is stained with the N-Histofine® (Nichirei, Japan) at room temperature and followed by treatment with substrate DAB chromogen (Novocastra/Leica Microsystems, IL, USA). The tissue biopsy is also counterstained with hematoxylin. The expression level of target proteins was evaluated according to the simplified H score system, which is based on the intensity of cell staining [3 (strong), 2 (moderate), 1 (weak), or 0 (no cell staining)] and the percentage of cell staining [3 (≥90%), 2 (50-89%), 1 (10-49%), or 0 (0-9%)]. The two scores were multiplied by each other and then divided by 3 to get the final score. Positive staining was defined as a final score ≥ 0.67.

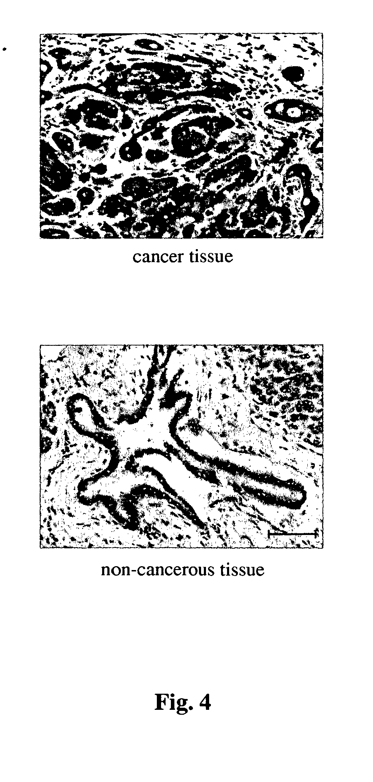

With reference to the Fig. 4, the immunohistochemistry assay of the cancer tissue biopsies from 67 pancreatic cancer patients shows that ULBP2 staining is positive in all biopsies (100%). Additionally, the expression of ULBP2 is more significant in the cancer tissue than in the adjacent non-cancerous tissues (the intensity of brown color indicates the expression level of the ULBP2). The mean expression level of the ULBP2 in the cancer tissue and in the adjacent non-cancerous tissue is score of 2.71±0.49 and 1.89±0.74, respectively. Moreover, with reference to Table 1, the expression of ULBP2 of the embodiment is not influenced by the clinically pathological symptoms such as the gender, age, histological grade, overall cancer status or TMN classification. The detecting result of the ULBP2 has highly consistence in patients with different clinically pathological symptoms.

**Table 1. Correlation between clinicopathological features and ULBP2 expression in 67 pancreatic cancer patients**

| Characteristics | Patient No. | IHC score (Mean ± SD)^{a} | *p*-value |
|---|---|---|---|
| Gender | | | |
| Male | 44 | 2.64 ± 0.54 | 0.180^{b} |
| Female | 23 | 2.83 ± 0.36 | |

| Age (years) | | | |
|---|---|---|---|
| < 64^{c} | 33 | 2.61 ± 0.55 | 0.089^{b} |
| ≥ 64 | 34 | 2.80 ± 0.41 | |

| Histological grade^{d} | | | |
|---|---|---|---|
| Well differentiation | 23 | 2.71 ± 0.59 | 0.746^{c} |
| Moderate differentiation | 31 | 2.68 ± 0.44 | |
| Poor differentiation | 10 | 2.70 ± 0.48 | |

| Overall stage | | | |
|---|---|---|---|
| stage I | 9 | 2.52 ± 0.82 | 0.593^{e} |
| stage II | 56 | 2.73 ± 0.43 | |
| stage IV | 2 | 3.00 ± 0.00 | |

| Tumor-node-metastasis (TNM)-T classification | | | |
|---|---|---|---|
| TNM-T2 | 9 | 2.52 ± 0.82 | 0.653^{b} |
| TNM-T3 | 58 | 2.74 ± 0.42 | |

| TNM-N classification | | | |
|---|---|---|---|
| TNM-N0 | 25 | 2.69 ± 0.59 | 0.806^{b} |
| TNM-N1 | 42 | 2.72 ± 0.43 | |

| TNM-M classification | | | |
|---|---|---|---|
| No metastasis | 65 | 2.70 ± 0.50 | 0.479^{b} |
| Distant metastasis | 2 | 3.00 ± 0.00 | |

| | | | |
|---|---|---|---|
| Intensity and percentage scores of cell staining were multiplied by each other and then divided by 3 to get the IHC scores. ^{b} By Wilcoxon test. ^{c} Median. ^{d} Histological grade information not available in 3 patients. ^{e} By Kruskal-Wallis test. | | | |

### Embodiment 3: the levels of the pancreatic cancer serological marker ULBP2 in serum sample of pancreatic cancer patients

A bead-based immunoassay is used to detect the ULBP2 level in a serum sample. An ULBP2 antibody, used as a capture antibody, is pre-coupled to COOH beads using the Bio-Plex Amine Couplin Kit (Bio-Rad). A biotin-conjugated anti-ULBP2 antibody is used as a detection antibody. The bead with the capture antibody is added in a filter-bottom 96-well microplate (Millipore). Then the serum sample solution or standard solution containing ULBP2 protein at various concentrations (3.91∼3.2× 10⁴ pg/mL) is added into the well to react in dark at room temperature for 1 hour. After washing the serum sample solution or the standard solution, the detection antibody is added into each well and reacted in dark at room temperature for 1 hour. After washing out the detection antibody, the phycoerythrin-conjugated streptavidin solution is added for 10 minutes to allow the binding between streptavidin and biotin. Unbound streptavidin is removed by a wash step. The ULBP2 level in the serum sample is then calculated by the fluorescent strength of the phycoerythrin based on the fluorescent strength of the standard calibration curve.

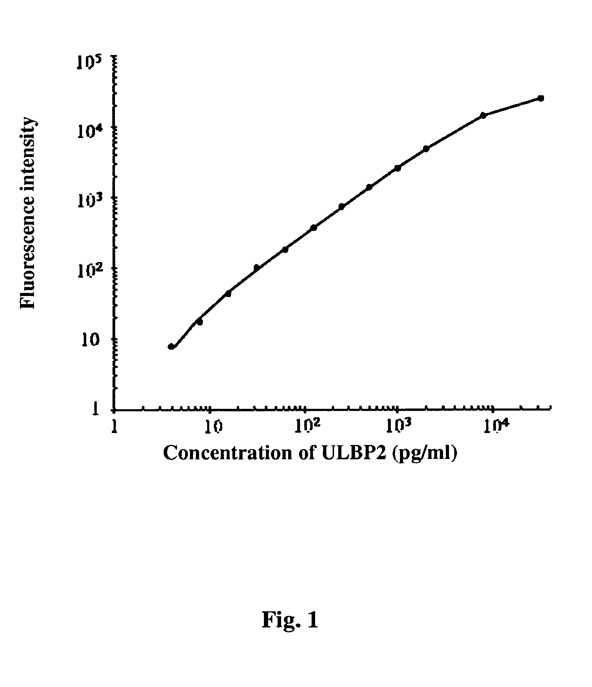

With reference to Fig.1, the ULBP2 in the serum sample is very easy to be detected using the bead-based immunoassay. The ULBP2 concentration ranged from 4.3 pg/mL to 31.9 ng/mL in the embodiment is precisely detected, which is not stably and accurately performed in a sandwich ELISA assay. The ULBP2 level is significantly increased in the cancer serum samples ( 200.2 ± 168.6 pg/ml) than in the healthy control samples (51.4 ± 64.6 pg/ml). When a cutoff value of 60 pg/mL for ULBP2 is chosen, the sensitivity and specificity values for cancer detection is 83.8% and 73.9%, respectively. These findings indicate that ULBP2 is a novel serum marker for pancreatic cancer detection. However, the serum ULBP2 levels are not statistically correlated with age, gender, histological grade, tumor overall stage, and TNM classification of pancreatic cancers in this case control study (Table 2).

**Table 2. Correlation of serum ULBP2 and CA19-9 with clinicopathologic characteristics in 154 pancreatic cancer patients.**

| Characteristics | No. | ULBP2 (pg/mL) Mean ± SD | *p*-value | CA 19-9 (U/mL) Mean ± SD | *p*-value |
|---|---|---|---|---|---|
| Gender | | | | | |
| Male | 111 | 199.5 ± 168.4 | | 64.7 ± 24.6 | |
| Female | 43 | 202.0 ± 170.8 | | 60.1 ± 23.7 | |

| Age (years) | | | | | |
|---|---|---|---|---|---|
| < 70^{b} | 75 | 200.4 ± 182.9 | | 60.2 ± 26.0 | |
| ≥ 70 | 79 | 199.9 ± 154.9 | | 66.4 ± 22.5 | |

| Histological grade^{c} | | | | | |
|---|---|---|---|---|---|
| Well differentiation | 12 | 148.8 ± 101.0 | 0.377^{d} | 70.0 ± 13.7 | 0.553^{d} |
| Moderate differentiation | 102 | 198.7 ± 173.1 | | 59.8 ± 26.0 | |
| Poor differentiation | 14 | 134.4 ± 99.9 | | 58.7 ± 25.2 | |

| Overall stage^{c} | | | | | |
|---|---|---|---|---|---|
| Stage I-II | 106 | 181.2± 158.8 | | 60.7 ± 24.4 | |
| Stage III-IV | 22 | 215.3 ± 178.5 | | 60.4 ± 28.9 | |

| Tumor-node-metastasis (TNM)-T classification^{c} | | | | | |
|---|---|---|---|---|---|
| TNM-T1 | 7 | 251.0 ± 150.0 | 0.418^{d} | 62.1 ± 20.5 | 0.536^{d} |
| TNM-T2 | 27 | 194.0 ± 192.9 | | 54.8 ± 28.4 | |
| TNM-T3 | 77 | 175.1 ± 150.5 | | 61.8 ± 24.0 | |
| TNM-T4 | 17 | 204.0 ± 171.1 | | 63.9 ± 26.8 | |

| TNM-N classification^{c} | | | | | |
|---|---|---|---|---|---|
| TNM-N0 | 57 | 191.6 ± 155.2 | | 62.1 ± 25.5 | |
| TNM-N1 | 71 | 183.4 ± 168.5 | | 59.4 ± 24.9 | |

| TNM-M classification^{c} | | | | | |
|---|---|---|---|---|---|
| No metastasis | 125 | 190.5 ± 162.4 | | 60.9 ± 25.2 | |
| Distant metastasis | 3 | 40.8 ± 27.7 | | 48.5 ± 22.1 | |

| | | | | | |
|---|---|---|---|---|---|
| ^{a} By Wilcoxon test. ^{b} Median. ^{c} Information of histological grade, overall stage and TNM stage not available in 26 patients. ^{d}By Kruskal-Wallis test. | | | | | |

The performance of the currently used pancreatic cancer marker CA 19-9 and the pancreatic cancer serological marker ULBP2 in 154 pancreatic cancer patients is compared to evaluate their detection efficacy. Both CA19-9 and ULBP2 show elevated serum levels in the pancreatic cancer patients than in the healthy controls and are not influenced by the clinicopathological characteristics. At 40 U/mL of CA 19-9, a cutoff value currently applied for pancreatic cancer screening in clinics, the sensitivity and specificity values is 84.4% and 74.6%, respectively. Noteworthily, upon selection of a cutoff value of 60 pg/mL for ULBP2, 21 of 24 pancreatic cancer patients with CA 19-9 levels < 40 U/mL could be discriminated form healthy individuals based on ULBP2 levels > 60 pg/mL. In addition, 24 of 36 healthy individuals with CA 19-9 levels > 40 U/mL could be further distinguished form the patients based on ULBP2 levels < 60 pg/mL. The combined usage of ULBP2 and CA19-9 has a great benefit to pancreatic cancer detection by using CA19-9 alone (shown in Table 3).

**Table 3. The efficacy of ULBP2 and CA 19-9 for detecting pancreatic cancers.**

| Cancer patients (n=154) | Total Sample | ULBP2 (> 60 pg/mL) | ULBP2 (< 60 pg/mL) |
|---|---|---|---|
| CA19-9 (< 40 U/mL) | 24 | 21 | 3 |
| CA19-9 (> 40 U/mL) | 130 | 108 | 22 |
| Healthy controls (n=142) | | | |
| CA19-9 (< 40 U/mL) | 118 | 24 | 82 |
| CA19-9 (> 40 U/mL) | 36 | 12 | 24 |

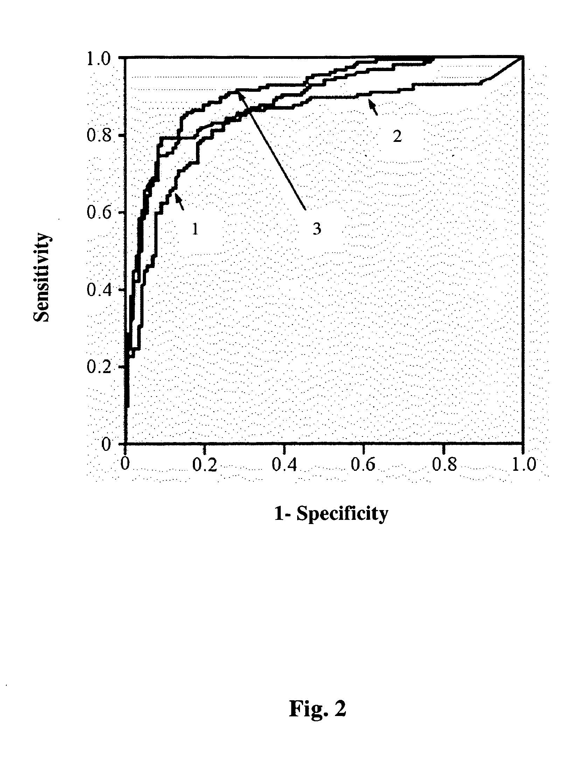

With reference to Fig. 2, the abilities of ULBP2 and CA 19-9 as detection markers are further tested by receiver operator characteristic (ROC) curve analysis and area under the ROC curve (AUC). The analysis demonstrated that ULBP2 (line 1) [AUC = 0.862, 95% confidence interval (CI), 0.821-0.904] is slightly better than CA 19-9 (line 2) (AUC = 0.856, 95% CI, 0.809-0.902) as a screening marker. Most importantly, the combination of ULBP2 and CA 19-9 (line 3) using the logistic regression model shows a higher diagnostic capacity than either marker alone (AUC = 0.910, 95% CI, 0.877-0.943). These results collectively reveal that ULBP2 is a useful serum marker for pancreatic cancer, especially when used together with CA 19-9.

### Embodiment 5: The ability of the pancreatic cancer serological marker ULBP2 for early detection of pancreatic cancer

142 healthy specimens and 154 pancreatic cancer patients are enrolled to evaluate the ability of ULBP2 for early detection of pancreatic cancers. The ULBP2 level in serum samples of the healthy controls is 51.4 ± 64.6 pg/mL that is less than that in the pancreatic cancer patients at any stage (TNM classification-T1/T2, TNM classification-N0 and overall Stage I-II is 205.7 ± 184.3 pg/mL, 191.6 ± 155.2 and 181.2 ± 158.8 pg/mL, respectively, p<0.0001). The results are similar to the CA19-9 and indicate the pancreatic cancer serological marker ULBP2 is able to use for early detection of pancreatic cancer.

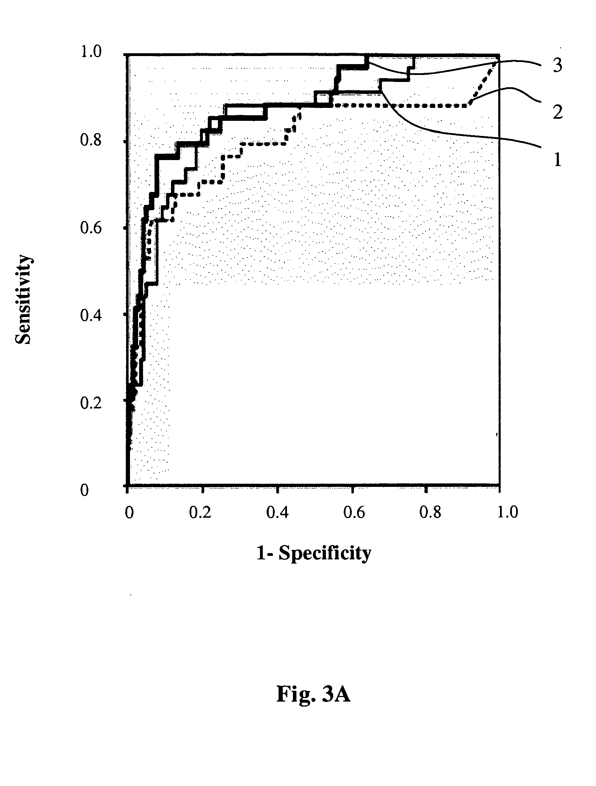

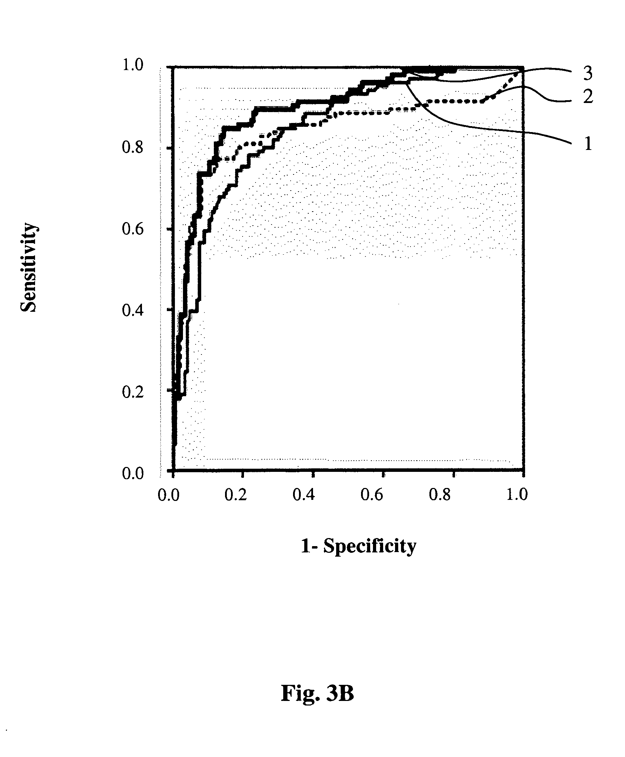

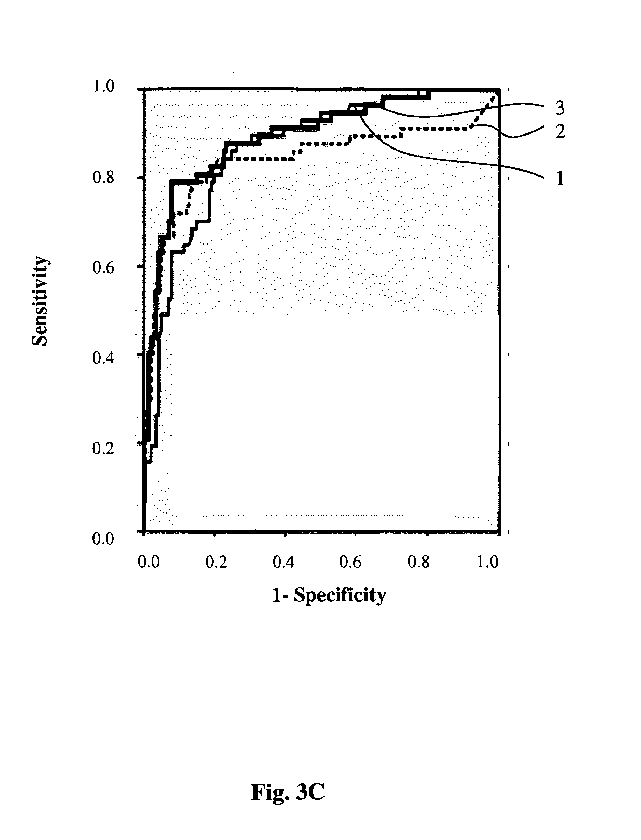

With reference to Figs. 3A to 3C, the ROC analysis shows that ULBP2 has better performance in early detection of pancreatic cancer than CA19-9. Furthermore, the detection efficiency is improved by combining ULBP2 and CA19-9

**Table 4. The ability of ULBP2 and CA 19-9 for early detection of pancreatic cancers**

| | ULBP2 | | CA 19-9 | | ULBP2 conbined with CA 19-9 | |
|---|---|---|---|---|---|---|
| | AUC | 95% CI | AUC | 95% CI | AUC | 95%CI |
| TNM classificati on-T1/T2 | 0.854 | 0.778∼0.930 | 0.796 | 0.690∼0.901 | 0.883 | 0.816∼0.949 |
| TNM classificati on-N0 | 0.866 | 0.811∼0.920 | 0.841 | 0.764∼0.917 | 0.893 | 0.841∼0.946 |
| Overall Stage I-II | 0.846 | 0.798∼0.895 | 0.839 | 0.782∼0.896 | 0.897 | 0.856∼0.937 |

### Embodiment 6: the specificity of the pancreatic cancer serological marker ULBP2

The specimens obtained from gastric cancer (GC), nasopharyngeal carcinoma cancer (NPC) and colorectal carcinoma cancer (CRC) patinets are applied to evaluate the specificity of the pancreatic cancer serological marker ULBP2. The ULBP2 levels in serum samples from the NPC and CRC, and plasma samples from the GC are detected. As shown in Table 5, compared with the healthy controls (51.4 ± 64.6 pg/mL for serum ULBP2), the serum ULBP2 levels are slightly higher in patients suffered from NPC (N = 28, 65.5 ± 74.3 pg/mL, p = 0.122) or CRC (N = 29, 70.6 ± 73.8 pg/mL, p = 0.038). However, the serum ULBP2 levels are significantly elevated in pancreatic cancer compared to that in CRC (200.2 ± 168.6 versus 70.6 ± 73.8 pg/mL, p < 0.0001) and NPC (200.2 ± 168.6 versus 65.5 ± 74.3 pg/mL, p < 0.0001). The results illustrate that ULBP2 represents a relative specific marker for pancreatic cancer, particularly that its level do not alter or only marginally elevated in the other two gastrointestinal cancers, CRC and GC.

**Table 5. The detection efficiency of the pancreatic cancer serological marker ULBP2 in different cancers.**

| | Sample No. | ULBP2 in serum (pg/mL) | ULBP2 in plasma (pg/mL) |
|---|---|---|---|
| Pancreatic cancer | 154 | 200.2±168.6 | - |
| GC | 30 | - | 78.1±79.7 |
| NPC | 28 | 65.5±74.4 | - |
| CRC | 29 | 70.6±73.8 | - |
| Control 1 | 142 | 51.4±64.6 | - |
| Control 2 | 25 | - | 86.1±101.2 |

Accordingly, the above-mentioned embodiments illustrate the serological marker ULBP2 is significant increased in the serum of a pancreatic cancer patient and is not correlative with the clinicophathological characteristics. The detection sensitivity of the serological marker ULBP2 is sharply improved to 3.91pg/mL in the serum sample. The serological marker ULBP2 has ability to detect the pancreatic cancer at early stage. The ULBP2 is combined with the CA19-9 to promote the efficiency and increase the specificity in pancreatic cancer detection, also in the early stage cancer detection. Therefore, the serological marker ULBP2 actually has capability to detect the pancreatic cancer in the early stage and strength the efficiency of the clinical diagnosis.

## Claims

1. A method for detecting pancreatic cancer, comprising steps of:
detecting: at least detecting the level of an UL16 binding protein 2 (ULBP2) having similarity of 95% or more with an amino acid sequence of SEQ ID No. 1 in a blood specimen, obtained from testee
calculating: calculating a concentration of the ULBP2 by using a stand and calibration curve and comparing the concentration of the ULBP2 with a control concentration of the ULBP2 of a blood specimen from a healthy person.

2. The method as claimed in claim 1, wherein the ULBP2 has an amino acid sequence of SEQ ID No. 1.

3. The method as claimed in claim 1, wherein the step of detecting further comprises at least additional serological marker of detecting a pancreatic cancer.

4. The method as claimed in claim 3, wherein the additional serological marker is carbohydrate antigen 19-9 (CA19-9).

5. The method as claimed in claim 1, wherein the blood specimen is a whole blood, a serum or a plasma blood specimen.

6. The method as claimed in claim 1, wherein the serological marker is applied in a bead-based immunoassay, a sandwich enzyme-linked immunosorbent assay (ELISA), a mass spectrometry-based assay or a mass spectrometry-based immunoassay.

7. The method as claimed in claim 2, wherein the serological marker is applied in a bead-based immunoassay, a sandwich enzyme-linked immunosorbent assay (ELISA), a mass spectrometry-based assay or a mass spectrometry-based immunoassay.

8. The method as claimed in claim 3, wherein the serological marker is applied in a bead-based immunoassay, a sandwich enzyme-linked immunosorbent assay (ELISA), a mass spectrometry-based assay or a mass spectrometry-based immunoassay.

9. The method as claimed in claim 4, wherein the serological marker is applied in a bead-based immunoassay, a sandwich enzyme-linked immunosorbent assay (ELISA), a mass spectrometry-based assay or a mass spectrometry-based immunoassay.

## Patentansprüche

1. Verfahren zur Erkennung von Bauchspeicheldrüsenkrebs, welches die folgenden Schritte umfasst:
Messung: in einer Blutprobe, die einer zu testenden Person entnommen wurde, wird zumindest die Menge eines UL16-bindenden Proteins 2 (ULBP2) ermittelt, welches eine 95%-ige oder höhere Übereinstimmung mit einer Aminosäuresequenz gemäß SEQ ID No. 1 aufweist,
Berechnung: unter Verwendung einer Standardkalibrationskurve wird die Konzentration von ULBP2 berechnet und diese Konzentration an ULBP2 mit einem Kontrollwert für die Konzentration an ULBP2 gemäß einer Blutprobe einer gesunden Person verglichen.

2. Verfahren nach Anspruch 1, wobei das ULBP2 eine Aminosäuresequenz gemäß SEQ ID No. 1 aufweist.

3. Verfahren nach Anspruch 1, wobei der Schritt der Messung außerdem wenigstens einen zusätzlichen serologischen Marker zur Erkennung von Bauchspeicheldrüsenkrebs umfasst.

4. Verfahren nach Anspruch 3, wobei der zusätzliche serologische Marker das Carbohydrat-Antigen 19-9 (CA19-9) ist.

5. Verfahren nach Anspruch 1, wobei die Blutprobe eine Vollblut-, eine Blutserum- oder eine Blutplasmaprobe ist.

6. Verfahren nach Anspruch 1, wobei der serologische Marker in einem Kügelchen - Immunoassay, einem Sandwich-ELISA (Enzyme - Linked - Immunosorbent - Assay), einer massenspektrometrischen Analyse oder einem massenspektrometrischen Immunoassay verwendet wird.

7. Verfahren nach Anspruch 2, wobei der serologische Marker in einem Kügelchen - Immunoassay, einem Sandwich - ELISA (Enzyme - Linked - Immunosorbent - Assay), einer massenspektrometrischen Analyse oder einem massenspektrometrischen Immunoassay verwendet wird.

8. Verfahren nach Anspruch 3, wobei der serologische Marker in einem Kügelchen - Immunoassay, einem Sandwich - ELISA (Enzyme - Linked - Immunosorbent - Assay), einer massenspektrometrischen Analyse oder einem massenspektrometrischen Immunoassay verwendet wird.

9. Verfahren nach Anspruch 4, wobei der serologische Marker in einem Kügelchen - Immunoassay, einem Sandwich - ELISA (Enzyme - Linked - Immunosorbent - Assay), einer massenspektrometrischen Analyse oder einem massenspektrometrischen Immunoassay verwendet wird.

## Revendications

1. Procédé de détection du cancer du pancréas comprenant les étapes de :
détection d'au moins le niveau d'une protéine 2 liant UL16 (ULBP2) ayant une similarité de 95% ou plus avec une séquence d'acides aminés de la SEQ ID No. 1 dans un échantillon de sang, obtenu d'un sujet à tester,
calcul d'une concentration de l'ULBP2 en utilisant une courbe d'étalonnage standard et comparaison de la concentration de l'ULBP2 avec une concentration de contrôle de l'ULBP2 d'un échantillon de sang d'une personne saine.

2. Procédé selon la revendication 1, l'ULBP2 ayant une séquence d'acides aminés de SEQ ID No. 1.

3. Procédé selon la revendication 1, l'étape de détection comprenant de plus au moins un marqueur sérologique supplémentaire de détection de cancer du pancréas.

4. Procédé selon la revendication 3, le marqueur sérologique supplémentaire étant l'antigène 19-9 d'hydrate de carbone (CA19-9).

5. Procédé selon la revendication 1, l'échantillon de sang étant un échantillon de sang total, de sérum ou de plasma sanguin.

6. Procédé selon la revendication 1, le marqueur sérologique étant appliqué dans un test immuno-sérologique à base de perles, un test d'immuno-absorption enzymatique en sandwich (ELISA), un test basé sur la spectrométrie de masse ou un test immuno-sérologique basé sur la spectrométrie de masse.

7. Procédé selon la revendication 2, le marqueur sérologique étant appliqué dans un test immuno-sérologique à base de perles, un test d'immuno-absorption enzymatique en sandwich (ELISA), un test basé sur la spectrométrie de masse ou un test immuno-sérologique basé sur la spectrométrie de masse.

8. Procédé selon la revendication 3, le marqueur sérologique étant appliqué dans un test immuno-sérologique à base de perles, un test d'immuno-absorption enzymatique en sandwich (ELISA), un test basé sur la spectrométrie de masse ou un test immuno-sérologique basé sur la spectrométrie de masse.

9. Procédé selon la revendication 4, le marqueur sérologique étant appliqué dans un test immuno-sérologique à base de perles, un test d'immuno-absorption enzymatique en sandwich (ELISA), un test basé sur la spectrométrie de masse ou un test immuno-sérologique basé sur la spectrométrie de masse.


### The sequence listing

<110> CHANG GUNG UNIVERSITY
<120> A Serological Marker for Detecting Pancreatic Cancer and A Method For Using The Serological Marker
<160> 1
<210> SEQ ID NO: 1
   <211> 240
   <212> NUCLEOTIDE
   <213> Homo sapiens
<400> 1

MetAlaAlaAlaAlaThrLysIleLeu LeuCysLeuProLeuLeuLeuLeuSer 20
GlyTrpSerArgAlaGlyArgAlaAspPro HisSerLeuCysTyrAspIleThrValIle 40
ProLysPheArgProGlyProArgTrpCys AlaValGlnGlyGlnValAspGluLysThr 60
PheLeuHisTyrAspCysGlyAsnLysThr ValThrProValSerProLeuGlyLysLys 80
LeuAsnValThrThrAlaTrpLysAlaGln AsnProValLeuArgGluValValAspIle 100
LeuThrGluGlnLeuArgAspIleGlnLeu GluAsnTyrThrProLysGluProLeuThr 120
LeuGlnAlaArgMetSerCysGluGlnLys AlaGluGlyHisSerSerGlySerTrpGln 140
PheSerPheAspGlyGlnIlePheLeuLeu PheAspSerGluLysArgMetTrpThrThr 160
ValHisProGlyAlaArgLysMetLysGlu LysTrpGluAsnAspLysValValAlaMet 180
SerPheHisTyrPheSerMetGlyAspCys IleGlyTrpLeuGluAspPheLeuMetGly 200
MetAspSerThrLeuGluProSerAlaGly AlaProLeuAlaMetSerSerGlyThrThr 220
GlnLeuArgAlaThrAlaThrThrLeuIle LeuCysCysLeuLeuIleIleLeuProCys 240